# EUROPEAN PATENT APPLICATION

(11) **EP 4 825 419 A1**
(43) Date of publication of application: **21.10.2026**
(21) Application number: 25305560.2
(22) Date of filing: 16.04.2025
(51) Int. Cl.: A61M 5/32

(54) **SAFETY DEVICE FOR A MEDICAL CONTAINER, INJECTION DEVICE INCLUDING THIS SAFETY DEVICE, AND METHOD FOR ASSEMBLING THE SAFETY DEVICE**

(71) Applicant: Becton Dickinson France, 38800 Le Pont-de-Claix (FR)
(72) Inventor: CARREL, Franck, 38220 SAINT-JEAN-DE-VAULX (FR); LLEWELLYN-HYDE, David, RG22 4WZ (GB); ALVAIN, Olivier, 38180 SEYSSINS (FR)
(74) Representative: Germain Maureau

(57) **Abstract**

This safety device (1) includes: a body (2) extending along a longitudinal axis A and configured for receiving a medical container (100) provided with an injection needle (103) covered by a needle shield (104); a needle guard (3) axially movable with respect to the body (2) between an initial position and a safety position distally located with respect to the initial position; a biasing member (4) for distally moving the needle guard (3) from the initial position to the safety position, the biasing member (4) defining an inner diameter (D3); a distal safety opening (3110) arranged at a distal end (311) of the needle guard (3); one or more safety walls (3111) extending through the distal safety opening (3110) for restricting access to an inner cavity of the needle guard (3) through the distal safety opening (3110); the one or more safety walls (3111) and the distal safety opening (3110) defining a first diameter (D1) configured for allowing passage of the needle shield (104) of the medical container (100) therethrough, and a second diameter (D2) greater than the first diameter (D1), the second diameter (D2) being configured for allowing passage of a guiding pin (5) configured for centering the biasing member (4) during assembly of the safety device (1).

## Description

The present invention relates to a safety device for a medical container, the safety device being configured for protecting a user against needle stick injuries after injection, an injection device including this safety device and this medical container, and a method for assembling the safety device or the injection device.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction away from the user's hand, and the "proximal direction" is to be understood as meaning the direction toward the user's hand.

Furthermore, in the present application, inner or inward means oriented towards a central longitudinal axis A, and outer or outward means oriented opposite to the central longitudinal axis A.

It is known to use safety devices for a medical container, the safety devices comprising a body for receiving the medical container, a guard for covering a used injection needle, a plunger rod to dispense medicine from the medical container and a biasing member for extension of the guard. The guard and the body may be held together by trigger fingers prior to use of the device.

Usually, the safety devices are designed to fit with syringes of 1 to 2,5 mL, which have a common predetermined diameter of typically 10,85 mm (the volume between 1 and 2,5 mL being set by the syringe length). However, some syringes are adapted to contain higher volumes of medical product, such as 3 mL to 5 mL syringes. These syringes have a larger diameter than the diameter of the 1 to 2,5 mL syringes. Typically, these syringes have a 14,45 mm diameter. This involves that the diameter of the safety device, and specifically the diameter of the guard of the safety device, needs to be larger to equip such large diameter syringes.

However, there is a risk that such a needle guard having an increased diameter guard no longer reliably prevents against needle stick injuries, and specifically no longer complies with the requirements of International Standard ISO 23908. International Standard ISO 23908 requires absence of contact between a used injection needle from a theoritecal ball having a diameter of 12 mm that would be tried to be inserted within a safety device. It is readily understandable that the larger the diameter of the guard is, the easier it is for the ball of 12 mm to be inserted within the guard, so that risks of contact between the used injection needle and the ball of 12 mm (which simulates a finger) is increased instead of being reduced. There is therefore a need for a safety device designed for big diameter syringes and which still provides reliable protection against needle stick injuries.

Another issue is the assembly of the body and the guard. This assembly requires the body to compress the biasing member within the needle guard. However, the biasing member may be axially longer than the guard. Besides, big diameter syringes may be axially shorter, and so may be guards of the safety devices equipping these big diameter syringes. This involves that, before assembly, a proximal portion of the biasing member may extend outside the guard. This "free" proximal portion may jeopardize assembly of the body and the guard, which requires compression of the biasing member whose proximal portion may bend in one or another direction instead of axially retracting within the guard. There is therefore a need for guiding the biasing member during assembly of the body and the guard. This guiding is a challenge, knowing that the distal opening of the needle guard needs to simultaneously satisfy with the above-mentioned safety requirements.

There is therefore a need for a safety device that alleviates some or all of the aforementioned drawbacks, and more specifically for a safety device that is designed for big diameter syringes, which still provides protection against needle stick injuries while providing a reliable and easy assembly.

In this context, an aspect of the invention is a safety device configured for protecting a user against needle stick injuries, the safety device including:
a body extending along a longitudinal axis A and configured for receiving a medical container provided with an injection needle covered by a needle shield,
a needle guard axially movable with respect to the body between an initial position and a safety position distally located with respect to the initial position,
a biasing member for distally moving the needle guard from the initial position to the safety position, the biasing member defining an inner diameter D3,
a distal safety opening arranged at a distal end of the needle guard,
one or more safety walls extending through the distal safety opening for restricting access to an inner cavity of the needle guard through the distal safety opening, the one or more safety walls and the distal safety opening defining
a first diameter D1 configured for allowing passage of the needle shield of the medical container therethrough,
a second diameter D2 greater than the first diameter D1, the second diameter D2 being configured for allowing passage of a guiding pin configured for centering the biasing member during assembly of the safety device.

The safety device of the invention therefore allows for ensuring reliable protection against needle stick injuries even with large diameter syringes which require the needle guard and the biasing member to be large enough for receiving the syringe.

The device of the invention may further include some or all of the features listed below.

In an embodiment, the first diameter D1 is defined between an inner edge of two diametrically opposite safety walls.

Advantageously, the second diameter D2 may be equal to or lower than the inner diameter D3 of the biasing member. The second diameter D2 may be slightly lower than the inner diameter D3.

Advantageously, the inner diameter D3 of the biasing member and the second diameter D2 of the distal safety opening are coaxial. They are defined with respect to the same central longitudinal axis. That is, the second diameter D2 coincides with, or is contained in, the inner diameter D3 when the safety device is seen from a bottom or top point of view, i.e. seen in the axial direction of the longitudinal axis A. The second diameter D2 and the inner diameter D3 may be both centered on the central longitudinal axis A.

In an embodiment, the distal safety opening includes one or more radial notches splitting the one or more safety walls.

In an embodiment, the second diameter D2 is defined between two diametrically opposite radial notches or between one of the one or more radial nocthes and an outer edge of one of the one or more safety walls.

In an embodiment, the one or more safety walls have an arc length greater than an arc length of the radial notches.

In an embodiment, the one or more safety walls include one or more guiding elements configured for guiding insertion of the needle shield of the medical container through the distal safety opening.

In an embodiment, the one or more guiding elements have an inner ramp configured for guiding the needle shield towards the distal safety opening.

In an embodiment, the one or more guiding elements have an outer ramp configured for guiding a distal end of the biasing member towards a proximal seat of the needle guard.

In an embodiment, the outer ramp is less inclined than the inner ramp with respect to the longitudinal axis A.

In an embodiment, the one or more guiding elements delimit a recess at a distal side of the one or more safety walls.

In an embodiment, the safety device includes the guiding pin, the guiding pin being configured for being inserted through the distal safety opening in order to center the biasing member during assembly of the safety device.

The guiding pin also guides compression of the biasing member during insertion of the body within the needle guard.

Advantageously, the guiding pin is configured to extend along the central longitudinal axis A to align the biasing member with the central longitudinal axis A during assembly of the safety device.

Advantageously, the guiding pin and the distal safety opening are complementarily shaped.

Advantageously, the guiding pin defines an outer diameter D2' equal to or lower than the second diameter D2. The outer diameter D2' may be slightly lower than the second diameter D2 and/or the inner diameter D3.

In an embodiment, the guiding pin includes one or more axial ribs configured for radially abutting against the biasing member to center the biasing member.

The diameter D2' of the guiding pin may be defined between a crest of two diametrically opposite axial ribs of the guiding pin.

In an embodiment, the one or more axial ribs of the guiding pin are configured to engage the one or more radial notches of the distal safety opening of the needle guard.

Advantageously, the guiding pin includes a central portion configured to extend between two diametrically opposite outer edges of the one or more safety walls.

That is, the central portion configured to extend within the first diameter D1 portion of the distal safety opening.

Advantageously, the central portion of the guiding pin has an outer diameter D1' equal to or lower than the first diameter D1 of the distal safety opening. The outer diameter D1' may be slightly lower than the first diameter D1.

Advantageously, the central portion includes a recessed proximal end.

Advantageously, the axial ribs include an inclined proximal end.

Another aspect of the invention is an injection device including a medical container arranged within the above-described safety device.

Another aspect of the invention is a method for assembling the aforementioned safety device, the method including the steps of:
- providing a body, a needle guard, a biasing member and a guiding pin;
- inserting the biasing member in the needle guard via an opened proximal end of the needle guard;
- inserting the guiding pin in the biasing member via the distal safety opening of the needle guard in order to center the biasing member;
- compressing the biasing member by inserting the body within the needle guard, until trigger fingers of the needle guard distally abut against the body to axially retain the needle guard in its initial position with respect to the body against the action of the biasing member.

Advantageously, the method includes the step of: inserting a medical container within the safety device. This permits to form the injection device.

Advantageously, the step of inserting the medical container within the safety device includes a step of centering the needle shield via guiding elements provided on the one or more safety walls of the needle guard.

The invention and the advantages arising therefrom will clearly emerge from the detailed description that is given below with reference to the appended drawings as follows :
- Figures 1A and 1B are, respectively, a perspective view and a cross-section view of an injection device according to an embodiment of the invention,
- Figure 2 is an exploded view of a safety device according to an embodiment of the invention,
- Figure 3 is a perspective view of a medical container of an injection device according to an embodiment of the invention,
- Figure 4 is a perspective view of a body of a safety device according to an embodiment of the invention,
- Figure 5A is a perspective view of a needle guard of a safety device according to an embodiment of the invention,
- Figure 5B is a bottom view of a needle guard of a safety device according to an embodiment of the invention,
- Figure 5C is a perspective cross-section view of a needle guard of a safety device according to an embodiment of the invention,
- Figure 5D is a cross-section view of a distal portion of a needle guard of a safety device according to an embodiment of the invention,
- Figure 5E illustrates an assembly step of an injection device according to an embodiment of the invention,
- Figure 6A illustrates the safety position of the needle guard of an injection device according to an embodiment of the invention,
- Figure 6B is a perspective bottom view of a distal portion of a needle guard of a safety device according to an embodiment of the invention,
- Figure 6C is a perspective cross-section view of a distal portion of a needle guard of a safety device according to an embodiment of the invention,
- Figure 7A illsutrates an assembly step of a safety device according to an embodiment of the invention,
- Figure 7B is a partial perspective view of a guiding pin for assembly of a safety device according to an embodiment of the invention,
- Figures 7C and 7B are, respectively, a side view and a cross-section view of an assembly step of a safety device according to an embodiment of the invention,
- Figure 8A is a perspective of a guiding pin for assembly of a safety device and an injection device according to an embodiment of the invention,
- Figure 8B is a cross-section view illustrating an assembly step of an injection device according to an embodiment of the invention.

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. In contrast, when an element is referred to as being "directly connected" or "directly coupled" to another element, there are no intervening elements present. Unless otherwise defined, all terms (including technical and scientific terms) used herein have the same meaning as commonly understood by one of ordinary skill in the art to which this disclosure belongs. It will be further understood that terms used herein should be interpreted as having a meaning consistent with their meaning in the context of this specification and the relevant art and will not be interpreted in an idealized or overly formal sense unless expressly so defined herein. Further, terms such as distal, proximal, up, down, bottom, and top are relative, and are to aid illustration, but are not limiting. Relative terms such as "below" or "above" or "upper" or "lower" or "horizontal" or "vertical" may be used herein to describe a relationship of one element to another element as illustrated in the Figures. It will be understood that these terms and those discussed above are intended to encompass different orientations of the device in addition to the orientation depicted in the Figures. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure. As used herein, the singular forms "a," "an," and "the" are intended to include the plural forms as well, unless the context clearly indicates otherwise. As used herein, the term "and/or" includes any and all combinations of one or more of the associated listed items. It will be understood that, although the terms first, second, etc., may be used herein to describe various elements, these elements should not be limited by these terms. These terms are only used to distinguish one element from another. For example, a first element could be termed a second element, and, similarly, a second element could be termed a first element without departing from the scope of the present disclosure. For simplification, the parts or elements of one embodiment which are found identically or similarly in the other embodiment will be identified using the same numerical references and will not be described again.

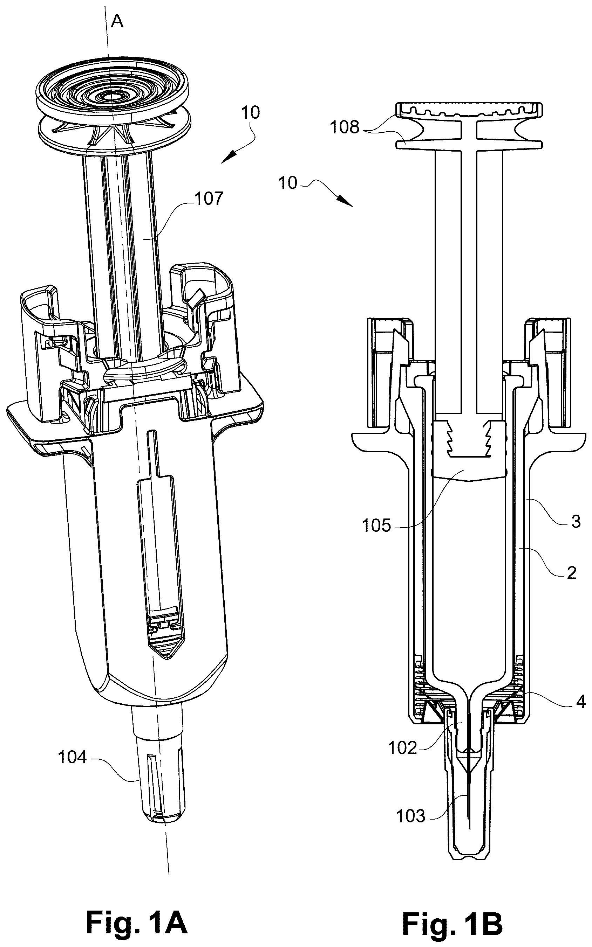

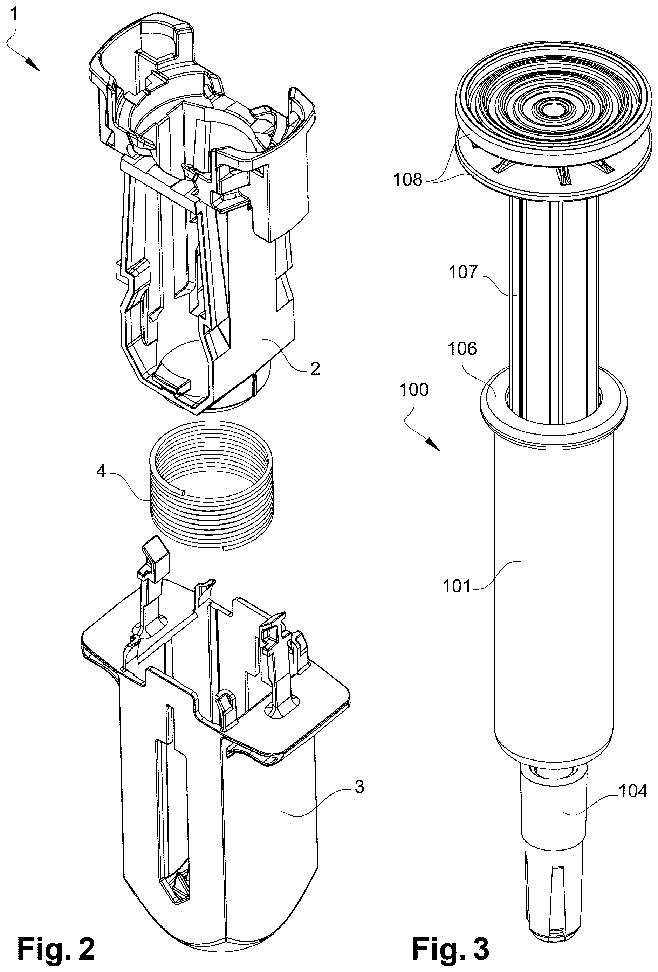

With reference to Figures 1A-1B is shown an injection device 10 according to an embodiment of the invention. The injection device 10 includes a safety device 1, Figure 2, and a medical container 100, Figure 3, received within the safety device 1. The safety device 1 is configured for protecting a user against needle stick injuries once injection is completed. The safety device 1 may include a body 2, a guard 3, and a biasing member 4.

As illustrated in Figure 1B or Figure 3, the medical container 100, such as a prefillable or prefilled syringe, may include a tubular barrel 101 defining a reservoir configured for containing a medical product. The tubular barrel 101 may include an elongated distal tip 102 defining an axial passageway for passage of the medical product from the reservoir and an injection needle 103 attached to this distal tip 102. Prior to use of the injection device 10, the injection needle 103 is sealed and protected by a removable needle shield 104 removably attached to the distal tip 102. A stopper 105 is arranged within the barrel 101 for pushing fluid outside the barrel 101 via the distal tip 102 and the injection needle 103. At its proximal end, the barrel 101 may include a radial flange 106. The barrel 101 may include a glass or a plastic material. The medical container 100 may include a plunger rod 107 for pushing the stopper 105 in the distal direction. The plunger rod 107 may include a flange or a double-flange 108 that may be arranged at a proximal end of the plunger rod 107 for allowing the user to apply his/her thumb on the flange or double-flange 108 in order to manually push the plunger rod 107 in the distal direction.

It is of note that the saftey device 1 is configured for cooperating with medical containers 100 such as big diameter syringes. By big diameter syringe it is meant that the barrel 101 of the medical container 100 has an outer diameter of 12 mm or more, preferably 14 mm or more, such as for instance a diameter of 14,45 mm more or less 0,1 mm. The barrel 101 may thus correspond to the barrel of a 3 mL to 5 mL syringe, although embodiments are not limited thereto, the volume of the barrel 101 possibly being less than 3 mL or more than 5 mL depending on the barrel axial length. The below described body 2, needle guard 3 and biasing member 4 are thus large enough, and specifically designed, to contain such a 3 mL or 5 mL syringe.

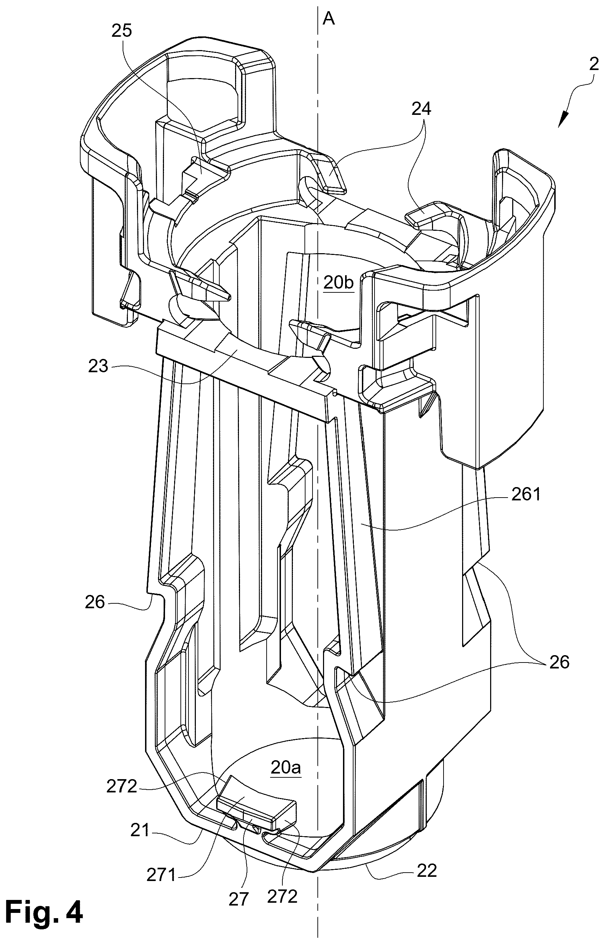

Figure 4 illustrates the body 2. As shown in Figure 1B, the body 2 is configured for receiving the medical container 100. The body 2 may be made of a single piece. The body 2 is arranged within the guard 3. The body 2 is proximal to the biasing member 4. The body 2 extends along the longitudinal axis A between an opened distal end 20a, configured for allowing passage of the distal tip 102 and the needle shield 104 of the medical container 100, and an opened proximal end 20b, configured for allowing insertion of the medical container 100 in the body 2. The opening delimited at the proximal end 20b, and/or the opening delimited at the distal end 20a, may be sized to allow passage of a medical container configured for containing 3 mL or more of the medical product, that is to allow passage of a barrel 101 having an external diameter equal to or greater than 12 mm or more, preferably 14 mm or more, such as for instance a diameter of 14,45 mm more or less 0,1 mm .

With reference to Figure 4, the body 2 may include some or all of a distal seat 21, a centering sleeve 22, a mounting proximal stop 23, one or more snap-fit members 24, one or more retaining proximal abutments 25, one or more safety distal stops 26, a proximal stop 271, and/or one or more guiding orthoradial abutments 272.

The distal seat 21 may be arranged at the distal end 20a of the body 2 for providing support to the biasing member 4. The centering sleeve 22 may be a cylindrical wall distally protruding from the distal end 20a of the body 2 and is configured to extend within the biasing member 4 to guide and center a proximal end of the biasing member 4. The distal seat 21 is proximal to the centering sleeve 22. The proximal stop 23 may be arranged at the proximal end 20b of the body 2 for axially abutting against a distal side of the flange 106 of the medical container 100 in order to block insertion of the medical container 100 within the body 2. The snap-fit members 24 may proximally protrude from the proximal end 20b of the body 2 for axially abutting a proximal side of the flange 106 of the medical container 100. Thus, the snap-fit members 24 cooperates with the proximal stop 23 to axially block the medical container 100 with respect to the body 2. The body 2 may for instance include four snap-fit members 24. The proximal abutment 25 is configured for axially abutting against trigger fingers of the needle guard 3. As long as the trigger fingers 32 of the guard 3 abut against the proximal abutments 25 of the body 2, the guard 3 is prevented from leaving its initial position. The safety distal stops 26 may be arranged in four recesses each configured for receiving a safety member 33 of the guard 3. The safety distal stops 26 axially abut against the safety members of needle guard 3 after injection when the needle guard 3 reaches its safety position so that the guard 3 is prevented from moving back in the proximal direction towards its initial position. The proximal stop 271 may be arranged at a proximal side of a guiding member 27 configured for sliding in a guiding window 34 of the guard 3. The proximal stop 271 is configured for stopping axial movement of the needle guard 3 when the needle guard 3 moves to the safety position. The proximal stops 271 thus act together with the safety distal stops 26 to axially lock the needle guard 3 in its safety position. The orthoradial guiding abutments 272 may be arranged at lateral sides of the guiding member 27 for guiding axial movement of the needle guard 3 with respect to the body 2.

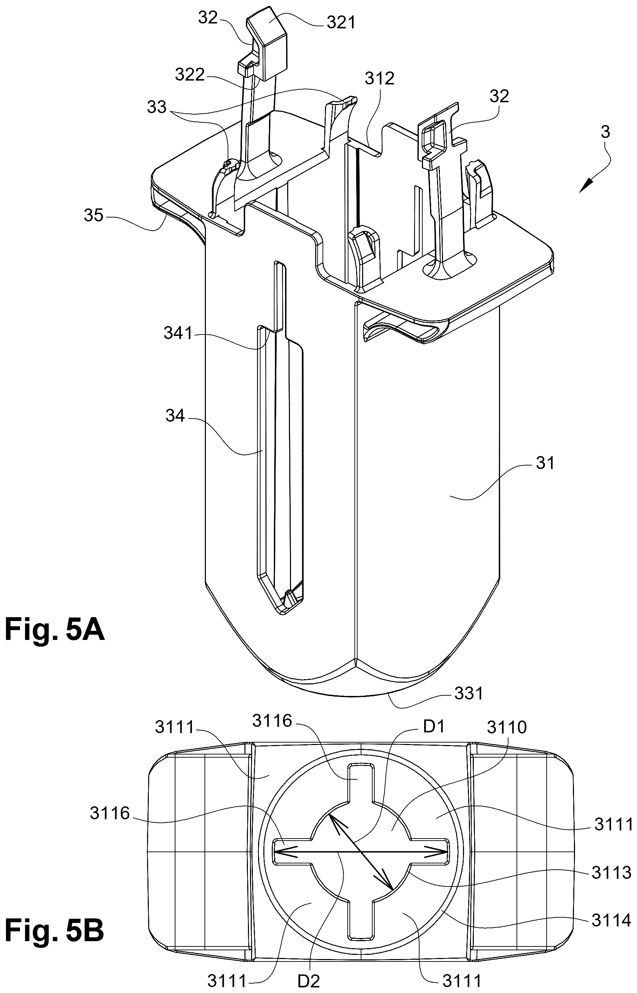

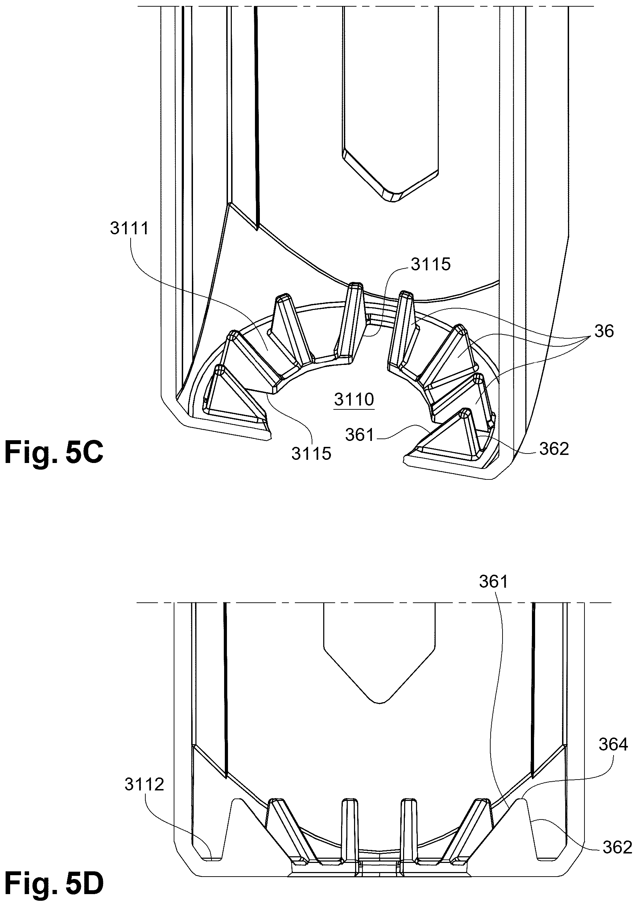

Figures 5A-5E illustrate the needle guard 3. The needle guard 3 is configured for extending around and distally beyond the injection needle 103 after injection to protect the user against needle stick injuries. The needle guard 3 may be made of a single piece.

The needle guard 3 may have a rectangular cross section shape. The needle guard 3 and the body 2 may be complementarily shaped, such that the body 2 extends within the needle guard 3. The needle guard 3 case has a tubular lateral wall 31, which defines an outer lateral surface and an opposite inner lateral surface, the inner lateral surface defining an inner cavity for accommodating the body 2.

The needle guard 3 is slidingly mounted onto the body 2. By means of its trigger fingers 32, the needle guard 3 is temporarily axially attached to the body. Before injection, the trigger fingers 32 maintain the needle guard 3 axially fixed with respect to the body 2.

The needle guard 3 longitudinally extends along the longitudinal axis A between an opened distal end 311, configured for allowing passage of a guiding pin 5 while restricting access to the injection needle 103, and an opened proximal end 312, configured for allowing insertion of the biasing member 4 and of the body 2 to assemble the safety device 1.

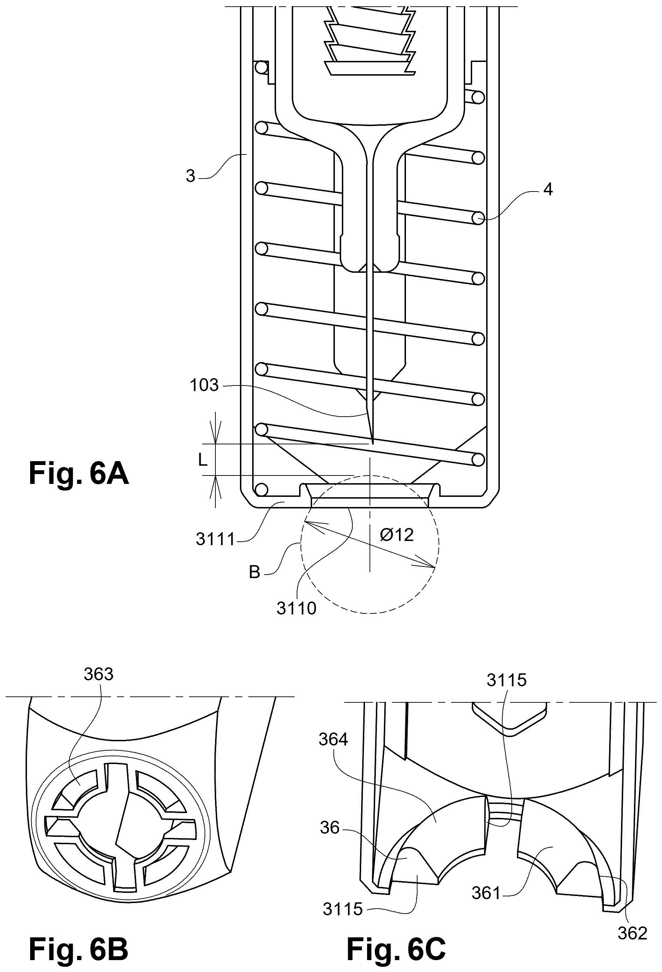

The distal end 311 defines a distal safety opening 3110 sized to forbid insertion of a 12 mm diameter ball within the needle guard 3, Figure 6A. The distal safety opening 3110 is sized so that there is no contact between the distal end of the injection needle 103 and the theoretical ball of 12 mm diameter. In the embodiment illustrated in Figure 6A, the ball penetrates no more than 2,1 mm within the needle guard 3. The distal safety opening 3110 thus has a predetermined diameter D1 configured for protecting against needle stick injuries. The predetermined diameter D1 is lower than 12 mm, and greater than an outer diameter of the needle shield 104 so that the needle shield 104 can extend through the distal safety opening 3110.

The distal end 311 includes a safety wall 3111 which may be orthogonal to the longitudinal axis A, and which extends radially inwardly towards the central longitudinal axis A. The safety wall 3111 defines the predetermined diameter D1. A proximal seat 3112 may be defined at a proximal side of the safety wall 3111 to provide support to the biasing member 4.

The needle guard 3 may include one or more, such as four, safety walls 3111, although embodiments are not limited thereto.

The safety walls 3111 may be in the form of ring portions, regularly distributed in a circumferential direction. The safety walls 3111 may include an inner peripheral edge 3113 fixedly connected to the needle guard 3 such that the safety walls 3111 are integral parts of the needle guard 3, and a free outer peripheral edge 3114. The diameter D1 may be defined between two diametrically opposite outer peripheral edges 3114.

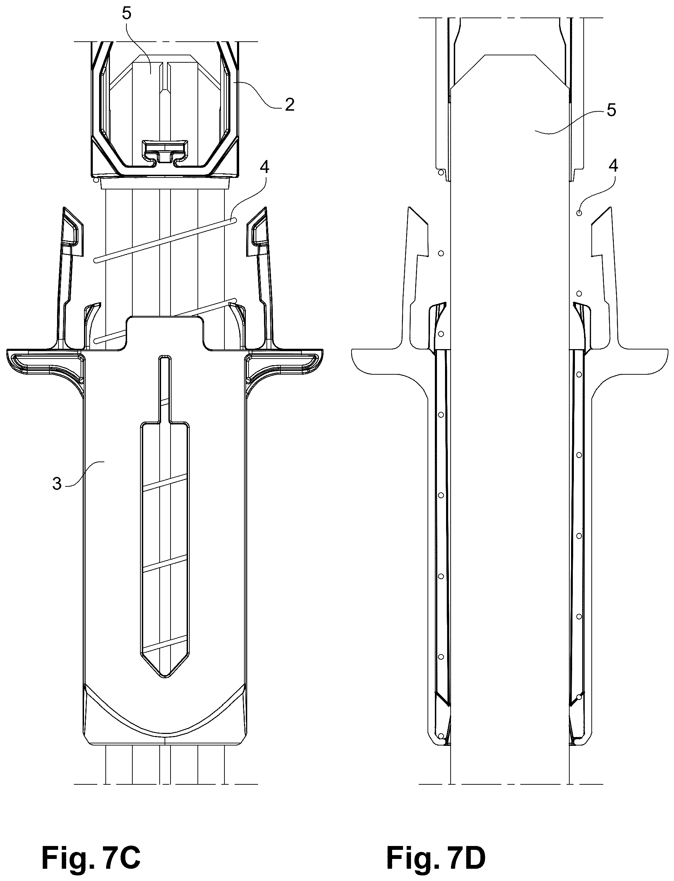

The needle guard 3 includes one or more radial notches 3116 that split or separate the one or more safety walls 3111. The radial notches 3116 are configured for allowing passage of a guiding pin 5 which is designed to center and guide the biasing member 4 when the body 2 is being assembled to the needle guard 3, Figures 7C-7D. The radial notches 3116 may define a diameter D2, Figure 5B, which is similar or slightly lower than an inner diameter D3 of the biasing member 4. The one or more radial notches 3116 and the safety walls 3111 therefore delimit a distal safety opening 3110 whose shape is complementary to the cross-section shape of the guiding pin 5. Therefore, the distal safety opening 3110 is a double diameter distal safety opening, including a safety diameter D1 protecting against needle stick injuries, and a mounting diameter D2 allowing centering and guiding of the biasing member 4 during assembly. The safety diameter D1 is lower than the mounting diameter D2.

The distal safety opening 3110 is a double-diameter opening. The first diameter D1 is a safety diameter, restricting access to the user, and the second diameter D2 is a guiding diameter, allowing easy, fast and proper assembly of the safety device 1. Thus, the distal safety opening 3110 includes a safety diameter D1 and a guiding diameter D2 which is greater than the safety diameter D2.

The distal safety opening 3110 may therefore have first diameter D1 portions (small diameter portions) and second diameter D2 portions (large diameter portions). The small diameter portions alternate with the large diameter portions in a circumferential direction. The small diameter portions correspond to the portions of the distal safety opening 3110 where the safety walls 3111 are located. The small diameter portions are circumferentially longer than the large diameter portions. That is, the safety walls 3111 are larger than the notches 3116 in a circumferential direction; their arc length is greater than the arc length of the notches 3116.

The needle guard 3 is moveable with respect to the body 2, specifically axially moveable with respect to the body 2, along the longitudinal axis A, between the following positions: an initial position, Figures 1A-1B, in which the needle guard 3 extends proximal to the injection needle 103 to allow for injection, and a safety position, Figure 6A, distally located with respect to the initial position, in which the needle guard 3 extends distal to the injection needle 103 to protect the user against needle stick injuries. Movement of the needle guard 3 from the initial position to the safety position is caused by the biasing member 4.

The biasing member 4, or safety spring, has a distal end 41 axially abutting against the proximal seat 3112 of the needle guard 3, a proximal end 42 axially abutting against the distal seat 21 of the body 2. The biasing member 4 extends between the distal end 20a of the body 2 and the distal end 311 of the needle guard 3. The biasing member 4 is thus located outside the body 2 and inside the needle guard 3. The biasing member 4 may be a coil spring defining an inner passage whose diameter D3 allows for extension of the medical container 100 therethrough. The diameter D3 may be equal to or greater than the diameter D2 of the distal safety opening 3110 of the needle guard 3.

In the initial position of the needle guard 3, the biasing member 4 is compressed. The trigger fingers 32 distally abut against the retaining proximal abutments 25 of the body 2. In the safety position of the needle guard 3, the safety members 33 of the needle guard 3 proximally abut against the safety distal stops 26 of the body 2 to prevent movement of the needle guard 3 back in the proximal direction.

The needle guard 3 may include some or all of: one or more trigger fingers 32, one or more safety members 33, one or more guiding windows 34, and/or one or more finger flanges 35 configured for providing support to the user's fingers.

The trigger fingers 32 may in the form of resiliently deformable legs proximally protruding from the proximal end 312 or finger flanges 35 of the needle guard 3 for retaining the guard 3 in its initial position against action of the biasing member 4. The trigger fingers 32 may therefore include a distal abutment 321 configured for axially abutting against the body 2 in the initial position of the needle guard 3. Opposite the distal abutment 321, the trigger fingers 32 may include a proximal ramp 322. The double flange 108 of the plunger rod 107 is configured for abutting against the proximal ramp 322 of the trigger fingers 32 at the end of injection to cause outward deformation of the trigger fingers 32, thereby moving the distal abutment 321 of the trigger fingers 32 out of contact with the retaining proximal abutments 25 of the body 2. Thus, the end of injection triggers deployment of the needle guard 3 from the initial position to the safety position.

The safety members 33 are configured for securing the needle guard 3 in its safety position with respect to the body 2. The safety members 33 may be resiliently deformable arms proximally protruding form the proximal end 312 of the needle guard 3 and configured for resiliently deflecting while sliding along a ramp 261 of the body 2, Figure 4, and shaped to engage a radial recess including the safety distal stop 26 when the needle guard 3 reaches the safety position.

The guiding windows 34 axially extend through the lateral wall 31 of the needle guard 3 and are configured for receiving the guiding member 27 of the body 2 to help guide axial movement of the needle guard 3 along the body 2. Besides, the distal stop 271 of the body 2 may be configured for axially abutting against a proximal end 341 of the guiding windows 34 to stop distal movement of the needle guard 3 with respect to the body 2.

The needle guard 3 may further include guiding elements 36 which may be arranged at the distal end 311 of the needle guard 3, for instance around the distal safety opening 3110, to guide and center the needle shield 104 when the medical container 100 is inserted within the safety device 1.

The guiding elements 36 may be integral with the needle guard 3. The guiding elements 36 may be in the form of axial protrusions proximally extending from a proximal side of the safety walls 3111. Each safety wall 3111 may include one or more guiding elements 36, such as three guiding elements 36 as illustrated in Figure 5C or a single guiding element 36 as illustrated in Figure 6C. As visible in Figure 5C, the guiding elements 36 may be regularly distributed along the safety wall 3111 in a circumferential direction; the guiding elements 36 may have a triangle shape. As visible in Figure 6C, the guiding element 36 may extend from one circumferential end 3115 of the safety wall 3111 to its opposite circumferential end 3115.

The guiding elements 36 may include some or all of an inner guiding ramp 361, an outer guiding ramp 362, and/or a recess 363.

The inner guiding ramp 361 is configured for guiding the needle shield 104 so that the needle shield 104 is centered with respect to the longitudinal axis A. The inner guiding ramp 361 is inclined with respect to the central longitudinal axis A, the inner guiding ramp 361 distally tapering from an apex 364 of the guiding element 36 towards the safety opening 3110. The outer guiding ramp 362 is arranged radially opposite the inner guiding ramp 361 and is configured for guiding insertion of the biasing member 4 within the needle guard 4. The outer guiding ramp 362 is inclined with respect to the longitudinal axis A and may be steeper than the inner guiding ramp 361. The recess 363, Figure 6B, extends within the guiding element 36 and forms a distal opening through a distal side of the safety wall 36.

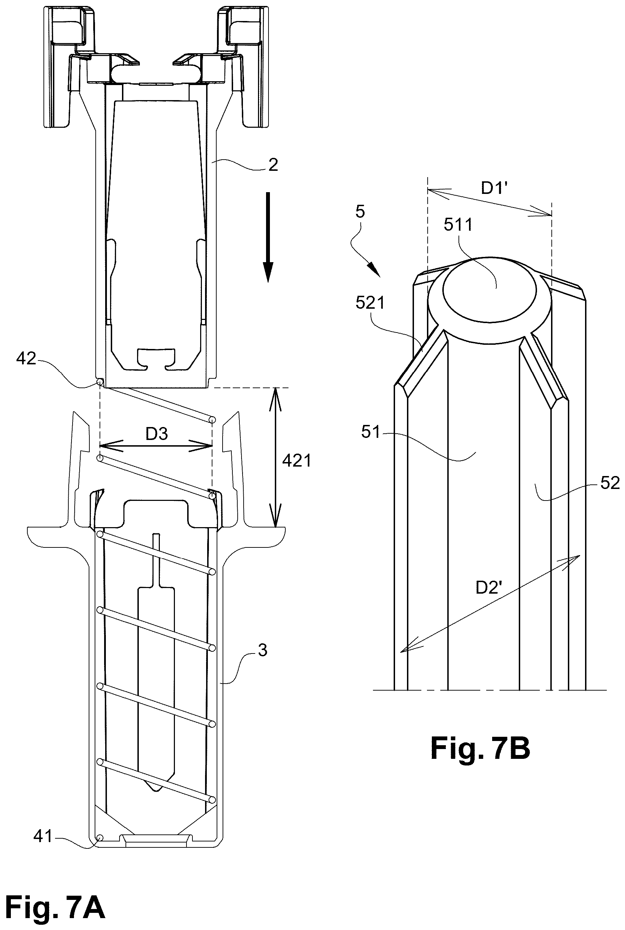

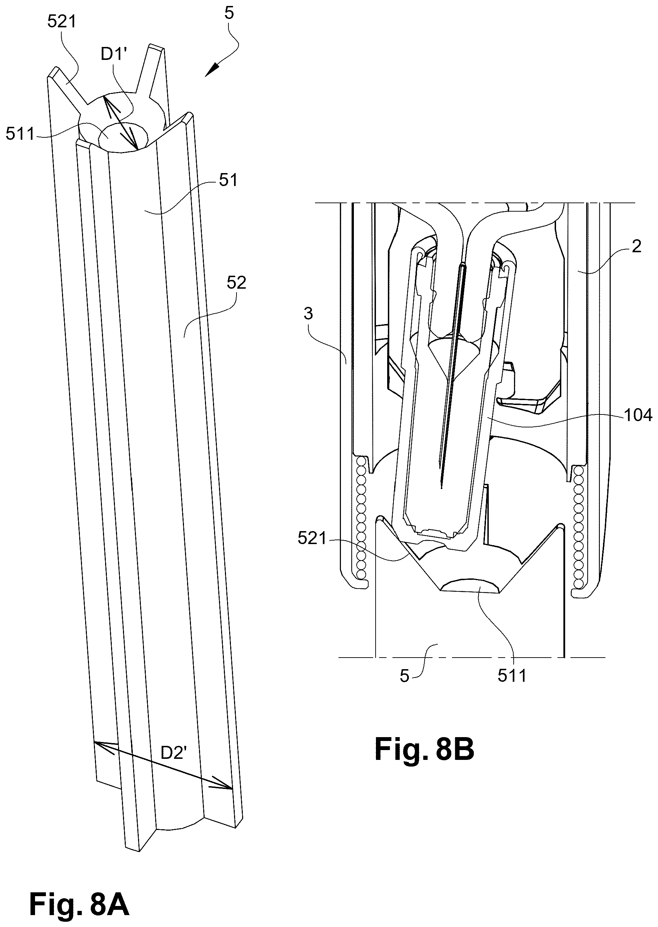

With reference to Figures 7B and 8A, the guiding pin 5 is a tool configured for guiding and centering the biasing member 4 during assembly of the safety device 1. The guiding pin 5 may be in the form of a straight elongated rod configured for being inserted through the distal safety opening 3110 of the needle guard 3, through the biasing member 4, and through a distal opening of the body 2. The guiding pin 5 may be longer than a total axial length of the needle guard 3. The guiding pin 5 may include a central portion 51 which may be cylindrical and which defines a diameter D1' which is equal to or lower than the diameter D1 of the distal safety opening 3110, and one or more protrusions outwardly radially protruding from an outer surface of this central portion 51. These protrusions may be in the form of axial ribs 52, which may be regularly distributed in a circumferential direction around the central portion 52. The axial ribs 52 define a diameter D2' which is equal to or lower than the diameter D2 of the notches 3116, and which may also be equal to or lower than the inner diameter D3 of the biasing member 4 such that the axial ribs 52 can center and guide the biasing member 4. For instance, the diameter D2' may be defined by the distance between the crest of two diametrically opposite axial ribs 52. There may be as many axial ribs 52 as notches 3116 between the safety walls 3111 of the needle guard 3. The axial ribs 52 and the notches 3116 may be complementarily shaped. The distal safety opening 3110 and the guiding pin 5 may have similar cross-section shapes and may thus be complementarily shaped so that the guiding pin 5 can engage the distal safety opening 3110 of the needle guard 3 to reach and guide the biaising member 4.

The central portion 51 may include a flat proximal end 511, Figure 7B, or a recessed proximal end 511, Figure 8A. The axial ribs 52 may have an inclined proximal end 521. In the embodiment of Figure 7B, the inclined proximal ends 521 proximally taper from the towards the central longitudinal axis A and the proximal end 511 of the central portion 51 to ease insertion of the guiding pin 5 through the needle gard 3 and the biasing member 4. In the embodiment of Figure 8A, the inclined proximal ends 521 distally taper towards the central longitudinal axis A and the proximal end 511 of the central portion 51 in order to guide and center the needle shield 104 when the medical container 100 is being placed within the safety device 1.

With reference to Figures 7A-7D and 8A-8B, a method for assembling the safety device 1 and the injection device 10 is detailed below.

The method may include a first step of providing the above-described body 2, needle guard 3, biasing member 4, and guiding pin 5. The biasing member 4 is inserted within the body 3, Figure 7A. A proximal portion 421 of the biasing member 4 may extend beyond the proximal end 312 of the guard 3. This free proximal portion 421 is thus not guided by the needle guard 3 when the body 2 is being inserted within the guard 3. This may cause the proximal portion 421 of the biasing member 4 to bend when compressed by the body 2. In order to avoid this, the method includes a step of proximally inserting the guiding pin 5 within the guard 3, the biasing member 4 and if need be the body 2, Figures 7C-7D, via the distal safety opening 3110 of the needle guard 3. Therefore, distal movement of the body 2 may be guided, and compression of the biasing member 4 may be guided. The body 2 is easily engaged within the needle guard 3 while compressing the biasing member 4. Insertion of the body 2 is complete when the trigger fingers 32 of the needle guard 3 axially abut against the body 2, thereby retaining the needle guard 3 in its initial position, axially fixed with respect to the body 2. The safety device 1 is assembled. The guiding pin 5 may be withdrawn or maintained as will be seen below for assembly of the injection device 10.

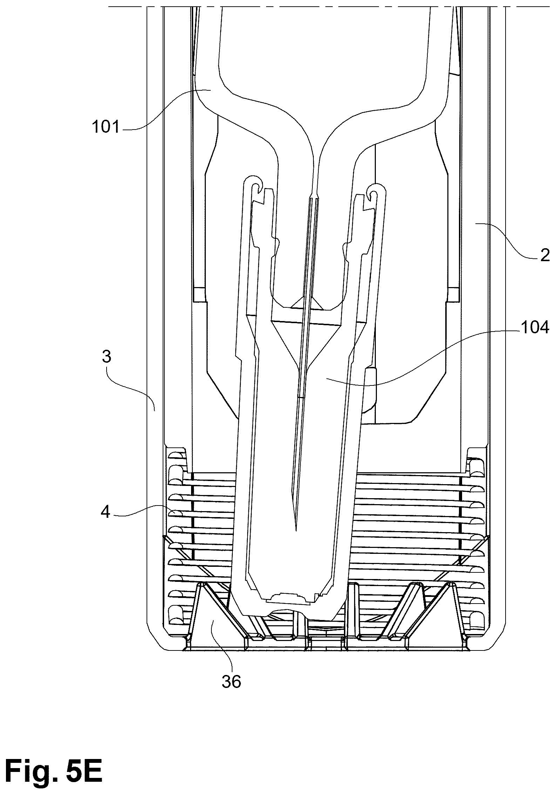

Assembly of the injection device 10 is obtained by insertion of the medical container 10 within the safety device 1. It is of note that due to manufacturing tolerances, the needle shield 104 and the barrel 101 of the medical container 100 may not be perfectly aligned. That is, the needle shield 104 may be inclined with respect to the distal tip 102 of the medical container 100 by an angle of 2° or less. It is also contemplated that the medical container 100 may not perfectly be aligned with the body 2 and the needle guard 3 when the medical container 100 is being inserted within the safety device 1. The method may therefore include a step of guiding and/or centering the medical container 100 by means of the needle shield 104 abutting against the guiding elements 36 of the needle guard 3, Figure 5E, during insertion of the medical container 100. In another embodiment, Figure 8B, the step of guiding and/or centering the medical container 100 may be performed by the guiding pin 5, when the needle shield 104 abuts against the proximal end 521 of the ribs 52. The guiding pin 5 may then be withdrawn.

In order to perform injection, the user removes the needle shield 104, makes the injection needle 103 penetrate into the injection site and pushes the plunger rod 107 in the distal direction to expel the medical product contained within the barrel 101. At the end of injection, the plunger rod 107 comes in abutment against the trigger fingers 32 and make them deflect radially outwardly. As a result, the needle guard 3 is no longer retained bay the body 2 and moves in the distal direction under the action of the biasing member 4 to cover the injection needle 103. As illustrated in Figure 6A, in the safety position, the safety walls 3111, and so the distal safety opening 3110, of the needle guard 3 ensures that a ball B of a predetermined diameter, such as a 12 mm diameter, cannot come into contact against the tip of injection needle 103. In the illustrated embodiment of Figure 6A, the safety walls 3111, and so the distal safety opening 3110, are configured such that the ball B and the tip of the injection needle 103 are separated by a predetermined axial distance L which may be equal to or greater than 1 or 2 mm, preferably 3 mm or 3,5 mm. The ball B may simulate a user's finger. Thus, after injection, the needle guard 3 efficiently protects the user against needle stick injuries, despite the larger diameter of the medical container 100 and hence of the needle guard 3.

It is readily understandable from the above description that the safety device 1 of the invention accordingly allows for reliable protection against needle stick injuries after injection, even when the medical container 100 is a big diameter syringe allowing injection of larger volumes of a medical product, such as 3 mL or more, for instance 5 mL or more. It is to be understood that the present invention is not limited to the embodiments described above and illustrated in the drawings; rather, the skilled person will recognize that many changes and modifications may be made within the scope of the appended claims.

## Claims

1. Safety device (1) configured for protecting a user against needle stick injuries, the safety device (1) including:
a body (2) extending along a longitudinal axis A and configured for receiving a medical container (100) provided with an injection needle (103) covered by a needle shield (104),
a needle guard (3) axially movable with respect to the body (2) between an initial position and a safety position distally located with respect to the initial position,
a biasing member (4) for distally moving the needle guard (3) from the initial position to the safety position, the biasing member (4) defining an inner diameter (D3),
a distal safety opening (3110) arranged at a distal end (311) of the needle guard (3),
one or more safety walls (3111) extending through the distal safety opening (3110) for restricting access to an inner cavity of the needle guard (3) through the distal safety opening (3110), the one or more safety walls (3111) and the distal safety opening (3110) defining
a first diameter (D1) configured for allowing passage of the needle shield (104) of the medical container (100) therethrough,
a second diameter (D2) greater than the first diameter (D1), the second diameter (D2) being configured for allowing passage of a guiding pin (5) configured for centering the biasing member (4) during assembly of the safety device (1).

2. Safety device (1) according to the preceding claim, wherein the first diameter (D1) is defined between an inner edge (3113) of two diametrically opposite safety walls (3111).

3. Safety device (1) according to the preceding claim, wherein the distal safety opening (3110) includes one or more radial notches (3116) splitting the one or more safety walls (3111).

4. Safety device (1) according to the preceding claim, wherein the second diameter (D2) is defined between two diametrically opposite radial notches (3116) or between one of the one or more radial nocthes (3116) and an outer edge (3114) of one of the one or more safety walls (3111).

5. Safety device (1) according to any one of the preceding claims, wherein the one or more safety walls (3111) have an arc length greater than an arc length of the radial notches (3116).

6. Safety device (1) according to any one of the preceding claims, wherein the one or more safety walls (3111) include one or more guiding elements (36) configured for guiding insertion of the needle shield (104) of the medical container (100) through the distal safety opening (3110).

7. Safety device (1) according to the preceding claim, wherein the one or more guiding elements (36) have an inner ramp (361) configured for guiding the needle shield (104) towards the distal safety opening (3110).

8. Safety device (1) according to any one of the preceding claims 6 or 7, wherein the one or more guiding elements (36) have an outer ramp (362) configured for guiding a distal end of the biasing member (4) towards a proximal seat (3112) of the needle guard (3).

9. Safety device (1) according to the preceding claim, wherein the outer ramp (362) is less inclined than the inner ramp (361) with respect to the longitudinal axis A.

10. Safety device (1) according to any one of the preceding claims 6 to 9, wherein the one or more guiding elements (36) delimit a recess (363) at a distal side of the one or more safety walls (3111).

11. Safety device (1) according to any one the preceding claims, wherein the safety device (1) includes the guiding pin (5), the guiding pin (5) being configured for being inserted through the distal safety opening (3110) in order to center the biasing member (4) during assembly of the safety device (1).

12. Safety device (1) according to the preceding claim, wherein the guiding pin (5) includes one or more axial ribs (52) configured for radially abutting against the biasing member (4) to center the biasing member (4).

13. Safety device (1) according to the preceding claim, wherein the one or more axial ribs (52) of the guiding pin (5) are configured to engage the one or more radial notches (3116) of the distal safety opening (3110) of the needle guard (3).

14. Injection device including a medical container (100) arranged within a safety device (1) according to any one the preceding claims.

15. Method for assembling a safety device (1) according to any one the preceding claims 1-13, the method including the steps of:
- providing a body (2), a needle guard (3), a biasing member (4) and a guiding pin (5);
- inserting the biasing member (4) in the needle guard (3) via an opened proximal end (312) of the needle guard (3);
- inserting the guiding pin (5) in the biasing member (4) via the distal safety opening (3110) of the needle guard (3) in order to center the biasing member (4);
- compressing the biasing member (4) by inserting the body (2) within the needle guard (3), until trigger fingers (32) of the needle guard (3) distally abut against the body (2) to axially retain the needle guard (3) in its initial position with respect to the body (2) against the action of the biasing member (4).
